# EUROPEAN PATENT APPLICATION

(11) **EP 3 603 627 A2**
(43) Date of publication of application: **05.02.2020**
(21) Application number: 18776381.8
(22) Date of filing: 28.03.2018
(51) Int. Cl.: A61K 31/047, A61K 9/06, A61K 47/10, A61K 47/32, A61K 47/38, A61P 15/02

(54) **LUBRICANT FORMULATIONS**

(30) Priority: 31.03.2017 ES 201700388
(71) Applicant: ITF Research Pharma, S.L.U, 28108 Alcobendas, Madrid (ES)
(72) Inventor: MOSCOSO DEL PRADO, Jaime, 28108 Alcobendas (ES); ESQUINAS GONZÁLEZ, Pedro Enrique, 28108 Alcobendas (ES)
(74) Representative: Linage González, Rafael
(86) International application number: PCT/ES2018/070266
(87) International publication number: WO 2018/178478

(57) **Abstract**

The invention relates to a water-based vaginal lubricant formulation containing at least one wetting agent, one lubricating agent, and one viscosifying agent for relieving symptoms associated with vaginal dryness.

## Description

### FIELD OF THE INVENTION

The present invention relates to vaginal lubricant formulations and the use thereof in the treatment of vaginal dryness.

### STATE OF THE ART

Although it is particularly common during and after menopause, vaginal dryness is prevalent among women of all ages. The reported values vary but it is estimated that about 15% of premenopausal women and up to 60% of postmenopausal women experience this condition.

In many women, symptoms associated with vaginal dryness have a negative impact on their quality of life, affecting their daily activities, mood, and sexual relations. Insufficient lubrication is a common complaint in women of all ages who, as a result, suffer from episodes of burning sensation, stinging, and vulvovaginal discomforts which can lead to pain during sexual activity (dyspareunia).

Vaginal dryness, also referred to as urogenital atrophy or atrophic vaginitis, is caused by a decrease in estrogen levels.

When the vagina is well supplied with estrogens, the epithelial tissue covering the genitourinary tract is thicker and has more folds, allowing it to stretch during sexual relations and labor. When estrogen levels drop, the vaginal mucosa becomes thinner and has fewer folds, causing it to become more fragile and less flexible. Furthermore, vaginal discharge, and therefore lubrication, are reduced. When this happens, the vulvovaginal epithelium is more prone to lesions and bleeding, and many women experience local symptoms (dryness, irritation, burning sensation, itchiness, and/or dyspareunia).

Estrogens levels naturally decrease after menopause or may increase as a result of the use of anti-estrogen drugs (for the treatment of breast cancer, endometriosis, uterine myomas, or infertility), removal of ovaries, radiotherapy of the pelvic area, chemotherapy, breastfeeding, stress, or excessive physical exercise.

The use of personal care products (soaps, lotions, perfumes, or vaginal douches), tobacco consumption, and the use of buffers or condoms may also damage the vulvovaginal epithelium.

First-line treatments for vaginal atrophy include non-hormonal vaginal lubricants and moisturizing creams. Lubricants have shown to be effective for rapid, short-term relief of vaginal dryness and dyspareunia. They must be applied frequently to provide more permanent relief, and re-applied before sexual relations in order to minimize friction and subsequent irritation.

Although vaginal lubricants have proven to be effective in relieving vaginal dryness, their components and/or properties can be a source of concern for the physician or patient in specific situations.

Lactobacilli constitute the predominant microbial flora in the vagina under normal conditions and play a critical role in the maintenance of the vaginal ecosystem by preventing the excessive proliferation of other common microorganisms, such as *Gardnerella vaginalis,* and hindering colonization by pathogenic microorganisms, such as *Escherichia coli.*

Therefore, it is highly recommended for vaginal lubricants not to affect local microbiota, given that dysbiosis of the vaginal cavity leads to the onset of vaginal infections (vaginosis).

Moreover, lubricant pH and osmolarity must be similar to those of the vagina under normal conditions, so as not to cause changes in or irritation to the vaginal epithelium.

The WHO recommends that lubricant formulation osmolarity not exceed 380 mOsm/kg. A higher osmolarity has been linked to a higher potential of causing mucosal irritation and vaginal epithelium damage. Despite this, the osmolarity of vaginal lubricants available on the market varies extensively, with many of them exceeding 1200 mOsm/kg.

The vaginal pH in women of childbearing age ranges between 3.8 and 5, depending on the phase of the menstrual cycle the women are in, while postmenopausal women have a vaginal pH of about 7. The pH of the different commercial lubricants varies within of a wide range. Studies conducted on animals suggest that values less than or equal to 3 would not be acceptable for human use.

As mentioned above, vaginal dryness is the most preferred pathology and it affects women of all ages.

For those women hoping to conceive, it is important that lubricant formulations do not affect sperm integrity and function, as this would reduce sperm fertilization potential. Despite this, several studies have reported a detrimental effect of lubricants available on the market on spermatozoid motility and function.

Finally, for women who are only looking to improve their sexual relations, it is important for the components of the lubricant formulation not to affect condom integrity.

Lubricants can be formulated based on water, silicone, or oily derivatives. Nevertheless, the use of oil-based formulations is often discouraged because they may damage latex condoms, in addition to being more difficult to wash out and favoring vaginal infections.

### SUMMARY OF THE INVENTION

There is therefore a need to have a vaginal lubricant formulation that brings together at the same time the following characteristics:
- does not alter normal vaginal flora,
- does not irritate vaginal mucosa,
- is compatible with condoms,
- maintains sperm viability.

The inventors of the present invention have found that a careful selection of the components thereof allows obtaining a lubricant formulation that meets all these requirements and, furthermore, exhibits organoleptic/physicochemical characteristics similar to those of normal vaginal secretions.

In particular, the inventors of the present invention have developed an aqueous formulation that brings together the following properties:
- pH: between 5.5 and 7.5, preferably between 6 and 7;
- viscosity: between 2000 and 4800 mPas, preferably between 2500 and 4500 mPas;
- osmolarity: less than or equal to 380 mOsm/kg, preferably between 300 and 380 mOsm/kg.

Accordingly, a first aspect of the present invention relates to a water-based vaginal lubricant formulation, comprising:
- a wetting agent;
- a lubricating agent;
- a viscosifying agent.

A second aspect of the present invention relates to the use of the vaginal lubricant formulation for relieving symptoms associated with vaginal dryness.

A third aspect of the present invention relates to the method for the preparation of the vaginal lubricant formulation.

### DRAWINGS

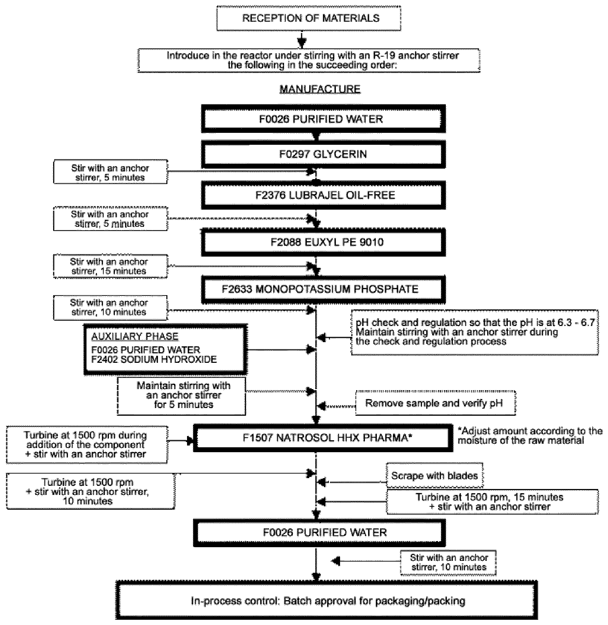

Figure 1 shows the diagram for the industrial-scale preparation of the lubricant formulation.

### DETAILED DESCRIPTION

In a preferred embodiment, the lubricant formulation is an aqueous gel comprising:
- a wetting agent selected from glycerin (or glycerol) and propanediol,
- a lubricating agent selected from glyceryl acrylate/acrylic acid copolymer and methylvinylether/maleic anhydride copolymer (PVM/MA copolymer), and mixtures thereof,
- a viscosifying agent selected from hydroxyethylcellulose (with different degrees of viscosity), xanthan gum, acacia gum, and mixtures thereof.

Preferably, the wetting agent is glycerin in an amount between 1 and 2% w/w, more preferably in an amount of (1.2 ± 0.1)% w/w.

Alternatively, it can contain propanediol in an amount between 1 and 3% w/w or a mixture of glycerin 1% w/w and propanediol 0.25% w/w as a wetting system.

Preferably, the lubricating agent is a mixture containing at least one glyceryl acrylate/acrylic acid copolymer in an amount between 0.005 and 0.007% w/w, and a methylvinylether/maleic anhydride copolymer in an amount between 0.0025 and 0.0035% w/w.

More preferably, the lubricant is a mixture of glyceryl acrylate/acrylic acid copolymer in an amount of (0.006 ± 0.0005)% w/w and PVM/MA copolymers in an amount of (0.003 ± 0.0002)% w/w.

In a particularly preferred manner, the lubricating agent is incorporated to the formulation as part of a lubricating system which furthermore includes a wetting agent and a preservative. For example, an amount of (0.5 ± 0.1)% by weight of the formulation of the lubricating system marketed under the brand Lubrajel Oil-Free®, containing glycerin (36.5 - 44.6% w/w), glyceryl acrylate/acrylic acid copolymer (1.07 - 1.31% w/w), PVM/MA copolymer (0.56 - 0.68% w/w), and phenoxyethanol (0.99 - 1.2% w/w), and water (50.8 - 60% w/w) can be used.

Preferably, the viscosifying agent is selected from hydroxyethylcellulose with different degree of viscosity, hydration, and biological and degradation resistance.

In a more preferred embodiment, the viscosifying agent is high-viscosity hydroxyethylcellulose (for example, hydroxyethylcellulose commercially available under the brand Natrosol 250HHX®), in an amount between 0.6 and 1% w/w, preferably in an amount of (0.8 ± 0.1)% w/w.

The lubricant formulation of the present invention can furthermore contain other excipients. Preferably, it furthermore contains at least one preservative agent and a pH regulating system (buffer).

The preservative agent is selected from phenoxyethanol, methylparaben sodium, propylparaben sodium, and mixtures thereof.

Additionally, it can contain a preservative agent enhancer selected from ethylhexylglycerin (or ethylhexylglycerol), phenethyl alcohol, phenylpropanol, and caprylyl glycol.

In a preferred embodiment, the formulation contains phenoxyethanol in an amount between 0.45 and 1% w/w, preferably in an amount of (0.9 ± 0.1)% w/w.

In another preferred embodiment, the formulation contains phenoxyethanol in an amount of (0.9 ± 0.1)% w/w and ethylhexylglycerin in an amount of (0.09 ± 0.01)% w/w.

Optionally, the preservative agent can be incorporated previously mixed with the enhancer. For example, an amount between 0.5 and 1% by weight of the formulation of the preservative system marketed under the brand Euxyl PE-9010®, containing phenoxyethanol (90% w/w) and ethylhexylglycerin (10% w/w), can be used.

The buffer is any pharmaceutically acceptable pH regulating system which allows maintaining the pH of the formulation between 6 and 7, preferably at a value of 6.5 ± 0.2.

Preferably, it is selected from potassium phosphate (monobasic potassium phosphate) buffer, citrate (trisodium citrate) buffer, and lactic (lactic acid) buffer, and prepared according to the European Pharmacopoeia.

In a preferred embodiment, the pH regulating system is formed by monobasic potassium phosphate in an amount between 0.65 and 0.70% w/w, more preferably in an amount of (0.68 ± 0.02)% w/w and 10% w/v sodium hydroxide in an amount between 0.2% and 1.2%, more preferably in an amount of (0.7 ± 0.1)% w/w.

The lubricant formulation of the present invention can furthermore contain other pharmaceutically acceptable excipients. In this sense, it may incorporate an ingredient with calming and/or regenerative properties, such as Biosaccharide Gum-1, a polysaccharide of galacturonic acid, L-fucose, and D-galactose, with skin hydration and calming properties (commercially available under the Fucocert® brand, for example) or Biosaccharide Gum-2, a polysaccharide of galacturonic acid, galactose, and rhamnose, which acts by reducing inflammatory reactions and promoting skin regeneration (marketed under the Rhamnasoft® brand, for example).

In a preferred embodiment, the lubricant formulation of the present invention brings together the following properties:
- pH: between 6 and 7, preferably 6.5 ± 0.2 (compatible with both external genitalia and spermatozoids as well as vaginal flora);
- viscosity: between 2500 and 4500 mPas, more preferably 3000 ± 500 mPas;
- osmolarity: between 300 and 380 mOsm/kg, preferably 365 ± 10 mOsm/kg.

Viscosity is measured with a rotary viscometer (Brookfield RVT or equivalent), with a spin of 4, at a speed of 20 rpm. The formulation must be at a temperature of 20±1ºC at the time of analysis.

Osmolarity is determined according to the European Pharmacopoeia and pH is determined using a pH meter.

The lubricant formulations of the present invention are useful for treating vulvovaginal symptoms associated with vaginal dryness, particularly dyspareunia, and for improving lubrication during sexual relations.

The lubricant formulations of the present invention can be prepared according to any method known by one skilled in the art.

In a particular embodiment, the lubricant formulation of the present invention is prepared according to the method described below.

The invention is illustrated with the following non-limiting examples.

### EXAMPLE 1: Preparation method

An amount of purified water between 85% and 90% of the total amount is added to a reactor.

All the water-soluble excipients (wetting agent, lubricant, preservative, and buffer) are then gradually added, one by one, under stirring, allowing sufficient time to elapse in each case for complete dissolution.

Once the water-soluble components are added, the pH of the solution is checked and adjusted in the established range of pH (6.3 - 6.7) to assure proper subsequent wetting of the viscosifying agent and to achieve the desired viscosity values.

When the pH of the solution has been adjusted to the pre-established value, the viscosifying agent is dispersed in the solution, adding it little by little and allowing sufficient time to elapse for its proper distribution and wetting.

Once the gel is formed, purified water is added until achieving the final weight of the formulation and the product is finally homogenized with a new stirring process of agitation.

Figure 1 schematically depicts the industrial-scale preparation method.

### EXAMPLE 2. Formulation A

| Ingredient | amount (g) |
|---|---|
| | |
| Glycerin | 1.203 g |
| Acrylic acid/glyceryl acrylate copolymer | 0.006 g |
| Methylvinylether/maleic anhydride copolymer | 0.003 g |
| Hydroxyethylcellulose | 0.800 g |
| Phenoxyethanol | 0.900 g |
| Ethylhexylglycerin | 0.099 g |
| Potassium phosphate | 0.680 g |
| 10% Sodium hydroxide | 0.700 g |
| Purified water | q.s.f 100 g |

### EXAMPLE 3. Formulation B

| Ingredient | amount (g) |
|---|---|
| | |
| Glycerin | 1.203 g |
| Acrylic acid/glyceryl acrylate copolymer | 0.006 g |
| Methylvinylether/maleic anhydride copolymer | 0.003 g |
| Hydroxyethylcellulose | 0.700 g |
| Phenoxyethanol | 0,800 g |
| Ethylhexylglycerin | 0.089 g |
| Potassium phosphate | 0.680 g |
| 10% Sodium hydroxide | 0.700 g |
| Purified water | q.s.f 100 g |

### EXAMPLE 4. Tests

### 1. NO VAGINAL FLORA ALTERATION:

A study to evaluate the compatibility of the vaginal lubricant formulation of the present invention with various lactobacillus strains, typical of vaginal flora, was conducted.

To that end, 0.1 ml of a suspension of 3 strains in saline solution (0.9% NaCl):
- *Lactobacillus acidophilus* CECT 362 4.3x10⁷ CFU/ml,
- *Lactobacillus jensenii* CECT 4306 5.7x10⁷ CFU/ml, and
- *Lactobacillus crispatus* CECT 4840 6.8x10⁷ CFU/ml,
were inoculated in 10 ml of different dilutions of Formulation B (Example 3) in saline solution (0.9% NaCl) :
- 100% Gel B: 1% bacterial suspension + 99% formulation B
- 80% Gel B: 1% bacterial suspension + 80% formulation B + 19% saline solution
- 60% Gel B: 1% bacterial suspension + 60% formulation B + 39% saline solution
- 40% Gel B: 1% bacterial suspension + 40% formulation B + 59% saline solution
- 20% Gel B: 1% bacterial suspension + 20% formulation B + 79% saline solution

The suspension of 3 lactobacillus strains was also inoculated in 10 ml of saline solution (without Formulation B as a negative control) and in 10 ml of a bactericidal solution (as a positive control).

All the samples were incubated for a pre-established contact time (30 minutes) at room temperature (20º-25ºC).

Once the contact time ended, lactobacillus count was performed by pouring in a plate.

The samples were stirred and 1 ml was taken from each sample. Initial dilution was performed in a neutralizer (peptone-lecithin-polysorbate broth) and subsequent dilutions were performed in a saline solution (0.9% NaCl).

One milliliter of each of the dilutions was seeded in TSA and incubated at 30º-35ºC.

Once the incubation time elapsed, count was performed.

| Formulation | Lactobacillus spp | Suspension control | Concentration | 100 % | | 80% | | 60% | | 40% | | 20% | |
|---|---|---|---|---|---|---|---|---|---|---|---|---|---|
| | | | Dilution | 10⁻² | 10⁻³ | 10⁻² | 10⁻³ | 10⁻² | 10⁻³ | 10⁻² | 10⁻³ | 10⁻² | 10⁻³ |
| Gel B | *L. crispatus* | 6.8 x 10⁵ | CFU/plate | 159 | 17 | >300 | 40 | >300 | 44 | >300 | 49 | >300 | 56 |
| | | | CFU/ml | 1.6 x 10⁴ | | 4.0 x 10⁴ | | 4.4x10⁴ | | 4.9x10⁴ | | 5.6x10⁴ | |
| | *L. acidophylus* | 4.3 x 10⁵ | CFU/plate | 132 | 18 | >300 | 77 | >300 | 84 | >300 | 96 | >300 | 85 |
| | | | CFU/ml | 1.3 x 10⁴ | | 7.7 x 10⁴ | | 8.4 x 10⁴ | | 9.6 x 10⁴ | | 8.5 x 10⁴ | |
| | *L. jensenii* | 5.7 x 10⁵ | CFU/plate | >300 | 48 | >300 | 40 | >300 | 43 | >300 | 192 | >300 | >300 |
| | | | CFU/ml | 4.8 x 10⁴ | | 4.0 x 10⁴ | | 4.3 x 10⁴ | | 1.9 x 10⁵ | | >3.0 x 10⁵ | |

| Formulation | Lactobacillus spp | Suspension control | Concentration | 100% | | 80% | | 60% | | 40% | | 20% | |
|---|---|---|---|---|---|---|---|---|---|---|---|---|---|
| | | | Dilution | 10⁻² | 10⁻³ | 10⁻² | 10⁻³ | 10⁻² | 10⁻³ | 10⁻² | 10⁻³ | 10⁻² | 10⁻³ |
| Bactericidal product | *L. crispatus* | 6.8 x 10⁵ | CFU/plate | 0 | 0 | 4 | 0 | 24 | 6 | >300 | 47 | >300 | 110 |
| | | | CFU/ml | <1.0 x 10² | | 4.0 x 10² | | 2.4 x 10³ | | 4.7 x 10⁴ | | 1.1 x 10⁵ | |
| | *L. acidophylus* | 4.3 x 10⁵ | CFU/plate | 0 | 0 | 0 | 0 | 6 | 0 | 19 | 2 | >300 | 84 |
| | | | CFU/ml | <1.0 x 10² | | <1.0 x 10² | | 6.0 x 10² | | 1.9 x 10³ | | 8.4 x 10⁴ | |
| | *L. jensenii* | 5.7 x 10⁵ | CFU/plate | 0 | 0 | 16 | 0 | 20 | 4 | >300 | 52 | >300 | 121 |
| | | | CFU/ml | <1.0 x 10² | | 1.6 x 10³ | | 2.0 x 10³ | | 5.2 x 10⁴ | | 1.2 x 10⁵ | |

The study allows concluding that there is a clear difference between the count obtained with the bactericidal product, showing a more than 3-log drop in the number of colonies, and the rest of the samples, showing no or almost no drop in the number of colonies.

### 2. NO VAGINAL MUCOSA IRRITATION:

The osmolarity of Formulation B (Example 3) was studied according to the European Pharmacopoeia with 4 different preservative concentrations:
- Gel B with 0.45% phenoxyethanol
- Gel B with 0.63% phenoxyethanol
- Gel B with 0.81% phenoxyethanol
- Gel B with 0.90% phenoxyethanol

A value below 380 mOsm/kg, the limit recommended by the WHO for minimizing the risk of epithelial damage that a personal lubricant may cause, was obtained in all cases. In particular, Formulation B with 0.81% phenoxyethanol and 0.90% phenoxyethanol showed an osmolarity of 365 mOsm/kg and 368 mOsm/kg, respectively.

According to the scientific bibliography relating to the effect of lubricant product osmolarity on the vaginal mucosa, the osmolarity values obtained with the formulation of the present invention have no negative effects on the vaginal epithelium.

### 3. COMPATIBILITY WITH CONDOMS:

The compatibility of the vaginal lubricant formulation of the present invention with both latex condoms and polyurethane condoms was studied.

To that end, the condoms were contacted with Formulation B (Example 3) and changes in the breaking and stress properties were evaluated according to the standard method (ASTM D7661-10).

Based on the results of the tests, it can be concluded that the formulation of the present invention shows complete compatibility with both types of condoms.

### 4. MAINTENANCE OF SPERM VIABILITY:

*In vitro* studies were conducted to evaluate the effect of the vaginal lubricant formulation of the present invention on human spermatozoid viability. To that end, the effect of the formulation on spermatozoid motility and vitality was analyzed.

First, semen samples from healthy donors were subjected to liquefaction at 37ºC for 30 minutes and they were analyzed to assure that their characteristics were within the reference limits established by the WHO.

Once liquefied, the total number of spermatozoids present in each of the samples was counted and they were then diluted in the different culture media: HTF + 10% HSA (human tubal fluid + 10% serum albumin, negative control), Octoxinol-9 spermicidal solution in HTF + 10% HSA (positive control), and 10% Gel B solution in HTF + 10% HSA.

The samples were incubated for 30 minutes at 37ºC and 5% CO₂.

Finally, the following studies were conducted with these samples:
- Motility study, which allows determining the percentage of spermatozoids with progressive motility (PR), non-progressive motility (NP), and immotility (IM) using a phase contrast microscope at x200 or x400.
- Vitality study by means of eosin-nigrosin, which allows counting the number of spermatozoids that are not stained (living) and stained (dead).
- Vitality test of by means of hypo-osmotic swelling, which allows counting the number of spermatozoids with a swollen membrane (living) and without a swollen membrane (dead).

It was concluded from the study that the presence of the formulation of the present invention:
- does not affect spermatozoid motility when compared with the motility in the negative control;
- does not significantly modify spermatozoid vitality measured using eosin-nigrosin when compared with the vitality observed in the negative control;
- does not significantly modify spermatozoid vitality determined by means of hypo-osmotic swelling when compared to the vitality observed in the negative control.

Furthermore, the osmolarity study serves to confirm the compatibility of the formulations of the present invention with spermatozoids, because spermatozoids (with an osmolarity of about 365 mOsm/kg) are practically isomolar with respect to the seminal plasma, the uterine, and the testicle fluid (with an osmolarity between 300 and 400 mOsm/kg).

## Claims

1. A vaginal lubricant composition, comprising:
- a wetting agent selected from glycerin and propanediol,
- a lubricating agent selected from glyceryl acrylate/acrylic acid copolymer and methylvinylether/maleic anhydride copolymer and mixtures thereof,
- a viscosifying agent selected from hydroxyethylcellulose of different degrees of viscosity, xanthan gum, acacia gum, and mixtures thereof.

2. The composition according to claim 1, **characterized in that** the wetting agent is glycerin in an amount between 1% and 2% w/w.

3. The composition according to claim 1, **characterized in that** the lubricating agent is a mixture containing a glyceryl acrylate/acrylic acid copolymer in an amount between 0.005% and 0.007% w/w, and a methylvinylether/maleic anhydride copolymer in an amount between 0.0025% and 0.0035% w/w.

4. The composition according to claim 1, **characterized in that** the viscosifying agent is high-viscosity hydroxyethylcellulose in an amount between 0.6% and 1% w/w.

5. The composition according to claim 1, **characterized in that** it further comprises a preservative agent selected from phenoxyethanol, methylparaben sodium, propylparaben sodium, and mixtures thereof.

6. The composition according to claim 5, wherein the preservative agent is phenoxyethanol in an amount between 0.45% and 1% w/w.

7. The composition according to claim 5, further comprising a preservative enhancer selected from ethylhexylglycerin, phenethyl alcohol, phenylpropanol, and caprylyl glycol.

8. The composition according to claims 6 and 7, wherein the preservative agent is a mixture of phenoxyethanol in an amount of (0.9 ± 0.1)% w/w and ethylhexylglycerin in an amount of (0.09 ± 0.01)% w/w.

9. The composition according to claim 1, **characterized in that** it further comprises a pH regulating agent selected from potassium phosphate buffer, citrate buffer, and lactic buffer.

10. The composition according to claim 9, wherein the pH regulating agent is potassium phosphate buffer formed by monobasic potassium phosphate in an amount between 0.65% and 0.70% w/w and 10% w/v sodium hydroxide in an amount between 0.2% and 1.2% w/w.

11. Use of the composition of any of the preceding claims for the treatment of vaginal dryness.

12. Use according to claim 11 for the treatment of dyspareunia.

13. Use of the composition of any of claims 1 to 10 for improving lubrication during sexual relations.
